# EUROPÄISCHE PATENTSCHRIFT

(11) **EP 2 137 294 B1**
(45) Veröffentlichungstag und Bekanntmachung des Hinweises auf die Patenterteilung: **17.05.2017**
(21) Anmeldenummer: 08715813.5
(22) Anmeldetag: 16.02.2008
(51) Int. Cl.: C12M 1/26, C12M 1/34, C12M 1/22

(54) **NÄHRMEDIENEINHEIT UND VERFAHREN ZUR AUFNAHME EINES FILTERS AUS EINER FILTRATIONSVORRICHTUNG**
NUTRIENT MEDIUM UNIT AND METHOD FOR HOLDING A FILTER FROM A FILTRATION DEVICE
UNITÉ DE MILIEU NUTRITIF ET PROCÉDÉ DE POSITIONNEMENT D'UN FILTRE D'UN DISPOSITIF DE FILTRATION

(30) Priorität: 21.03.2007 DE 102007014081; 24.01.2008 DE 102008005968
(43) Veröffentlichungstag der Anmeldung: 30.12.2009
(73) Patentinhaber: Sartorius Stedim Biotech GmbH, 37079 Göttingen (DE)
(72) Erfinder: PFLANZ, Karl, 37130 Gleichen (DE)
(86) Internationale Anmeldenummer: PCT/EP2008/001213
(87) Internationale Veröffentlichungsnummer: WO 2008/113444

(56) Entgegenhaltungen:
- WO-A-00/17315
- DE-B3-102005 008 220
- US-A- 4 299 921
- US-A- 4 668 633
- US-A- 5 139 951
- US-A- 5 958 762
- US-A1- 2002 096 468
- US-A1- 2004 209 349
- US-A1- 2007 175 897

## Beschreibung

### Gebiet der Erfindung

Die Erfindung betrifft eine Nährmedieneinheit zur Aufnahme eines Filters einer Filtrationsvorrichtung mit einem mit Nährmedium gefüllten Unterteil und einem Deckel.

Weiterhin betrifft die Erfindung ein Verfahren zur mikrobiologischen Untersuchung flüssiger Proben nach vorangegangener Filtration, bei dem ein Membranfilter von einer Filterunterstützung abgehoben und auf einer Oberfläche eines in einem Unterteil einer Nährmedieneinheit angeordneten Nährbodens abgelegt und das Unterteil von einem Deckel abgedeckt wird.

Zur mikrobiologischen Analyse von zu filtrierenden Flüssigkeiten bzw. Proben mit einem Membranfilter wird nach der Filtration der Filter entnommen und beispielsweise in Petrischalen mit einem Nährboden aus Agar eingelegt und mit einem Deckel abgedeckt, wobei die Nährmedieneinheit in einem Brutschrank mehrere Tage bei erhöhter Temperatur lagert. Über den Nährboden erhalten die eventuell vorliegenden Mikroorganismen Nährstoffe, die zum Wachstum anregen, sodass diese bestimmt oder gezählt werden können.

### Stand der Technik

So ist beispielsweise aus der DE 10 2005 008 220 B3 eine Filtrationsvorrichtung mit einem auf einer Filterunterstützung eines Vorrichtungsunterteiles angeordneten Membranfilter und einem auf das Vorrichtungsunterteil aufsetzbaren Aufsatz bekannt.

Nachteilig bei dieser Vorrichtung, die sich grundsätzlich bewährt hat, ist, dass ein spezieller aufspreizbarer Entnahmering notwendig ist, mit dem der Membranfilter entnommen und in ein schalenförmiges Unterteil der Nährmedieneinheit abgelegt wird.

Weiterhin ist aus der DE 198 23 993 B4 eine Einwegvorrichtung zur Keimzahlbestimmung in Flüssigkeiten mit einem Aufgusstrichter, einer mikroporösen Membran mit Membranträger und einer Membranunterstützungseinheit bekannt, wobei der Aufgusstrichter und der Membranträger miteinander fixierbar sind und wobei am Ausgusstrichter mindestens eine Auswerfvorrichtung für den Membranträger vorgesehen ist und der Membranträger und die Auswerfvorrichtung von einer Membranunterstützungseinheit lösbar übergriffen werden.

Nachteilig dabei ist, dass die Filtrationseinheit relativ aufwendig ausgebildet ist und nach der Filtration in einem separaten Schritt über einen definierten Druckpunkt die Filterunterstützung gelöst werden muss. Danach muss in einem zweiten zusätzlichen Schritt die Aufgusstrichter/ Membranträgereinheit über dem geöffneten Unterteil der Nährmedieneinheit, d.h. der Agarschale, positioniert und mittels eines zweiten und um 90° versetzten Druckpunktes die Membran auf dem Nährboden abgelegt werden.

Weiterhin ist aus der EP 0 150 775 B1 eine Vorrichtung für die Untersuchung einer Flüssigkeitsprobe mittels Membranfiltration bekannt, bei der der Membranfilter dichtend am Ende einer Hülse befestigt ist. Dieses Hülsenende ist so ausgebildet, dass es als Einsteckaufnahme für einen Medienbehälter dient. Das zweite Hülsenende kann mit einem Deckel dicht verschlossen werden, um die Membran-Medieneinheit verschlossen in den Brutschrank zu geben.

Nachteilig dabei ist eine feste Verbindung zum Aufgusstrichter, die erfordert, dass entweder ein großes Totvolumen akzeptiert wird oder aber zusätzliche Handhabungsschritte zum Verringern des Totvolumens durch Stauchen oder aber auch zum Trennen der Einheit notwendig sind.

Weiterhin ist aus der US 2002/0096468 A1 eine Nährmedieneinheit bekannt, die aus Teilen einer Filtrationsvorrichtung zusammengesetzt wird. Nach einem Filtrationsvorgang wird ein Deckel und ein Aufsatz bzw. Trichter von einem Vorrichtungsunterteil, auf dem auf einer als Kissen ausgebildeten Filterunterlage ein Filter angeordnet ist, abgenommen. Der Filter ist dabei mit einem Fixierrand, der als Dichtung wirkt, an dem Vorrichtungsunterteil permanent verklebt. Der Deckel des Aufsatzes wird auf das Vorrichtungsunterteil der Filtrationsvorrichtung nunmehr direkt aufgesetzt und Vorrichtungsunterteil und Deckel werden mit dem Filter gewendet, so dass der Deckel ein Unterteil bildet. Anschließend wird über den Abfluss des Vorrichtungsunterteils Nährlösung zugeführt und der Abfluss mit einem speziellen Stopfen verschlossen. Erst dann kann die Vorrichtung in einen Inkubator eingebracht werden.

Nachteilig bei der bekannten Vorrichtung ist, dass die Filtrationsvorrichtung durch die Vielzahl ihrer Teile relativ kostenintensiv ist und das Vorrichtungsunterteil zumindest während einer Inkubationszeit im Brutschrank für eine weitere Filtration nicht zur Verfügung steht. Soweit der Filter im Vorrichtungsunterteil verklebt ist, kann die Filterunterlage nicht ausgetauscht, sondern lediglich mit einer Nährlösung relativ umständlich getränkt werden. Bei dieser bekannten Vorrichtung verbleibt der Filter also entweder in dem Unterteil der Filtrationsvorrichtung oder der Filter wird in bekannter Weise mit einem Werkzeug bspw. einer Pinzette entnommen.

Aus der US 4 299 921 A ist eine Nährmedieneinheit bekannt, die zwischen ihrem Unterteil und ihrem Deckel eine Dichtung aufweist. Die Dichtung ist entweder auf dem Rand des Unterteils oder im Deckel über eine Klebeschicht befestigt.

Nachteilig bei dieser bekannten Nährmedieneinheit ist, dass ein Filter aus einer Filtrationsvorrichtung mit bspw. einer Pinzette eingebracht werden muss.

### Aufgabenstellung

Aufgabe der vorliegenden Erfindung ist es daher, eine Vorrichtung und ein Verfahren bereitzustellen, bei dem es möglich ist, den Filter nach der Filtration ohne komplizierte Filtrationsvorrichtung und ohne den Gebrauch eines zusätzlichen Hilfsmittels einfach und kostengünstig in die Nährmedieneinheit einzubringen.

Die die Vorrichtung bzw. Nährmedieneinheit betreffende Aufgabe wird in Verbindung mit dem Oberbegriff des Anspruches 1 dadurch gelöst, dass der Deckel einen in das Unterteil hineinragenden Fixierrand aufweist, der zur Entnahme des Filters aus der Filtrationsvorrichtung mit einem Rand des Filters über eine Klebehaftung mit dem Filter verbindbar ist.

Dadurch, dass der Deckel einen Fixierrand aufweist, der auf einen korrespondierenden Rand des Filters über eine Klebehaftung aufsetzbar ist, haftet der Filter am Deckel und lässt sich leicht und einfach von der Filterunterstützung abheben und in das Unterteil der Nährmedieneinheit einbringen. Dabei können zum einen herkömmliche unkomplizierte Filtrationsvorrichtungen verwendet und zum anderen kann ohne zusätzliches Werkzeug der Filter in die Nährmedieneinheit eingebracht werden.

Nach einer bevorzugten Ausführungsform der Erfindung ist die Klebehaftung temporär oder reversibel ausgebildet. Dadurch kann nach einer Inkubation der Deckel von dem Filter leicht entfernt bzw. abgehoben werden.

Die Klebehaftung ist gemäß einer bevorzugten Ausführungsform der Erfindung als eine Haftschicht aus einem geeigneten Kleber ausgebildet und kann am Fixierrand des Deckels oder am korrespondierenden Rand des Filters angeordnet sein. Die Kraft der Klebehaftung kann so gewählt werden, dass ein nachträgliches Untersuchen der Probe nach Inkubation möglich ist, indem man den Filter wieder von dem Nährboden bzw. dem Fixierrand des Deckels abzieht und somit Zugang zu den gebildeten mikrobiologischen Kolonien hat.

Nach einer weiteren bevorzugten Ausführungsform der Erfindung ist die Haftschicht aus PSA-Dispersionsklebstoff oder Acrylat-Copolymer-Mikrokugeln ausgebildet. Dadurch sind auch nasse Filter ausreichend am Fixierrand des Deckels anhaftbar und auch wieder abziehbar. Geeignete druckempfindliche Kleber sind dem Fachmann beispielsweise als mikrokugelbasierte Acrylatkleber bekannt.

Gemäß einer weiteren bevorzugten Ausführungsform ist die Haftschicht dergestalt ausgeführt, dass sie für die Abnahme des Filters von der Filtrationsvorrichtung eine möglichst hohe Klebekraft aufweist und nach dem Kontakt mit dem feuchten Filter und dem feuchten Nährmedium zeitversetzt wieder lösbar ist und die Klebekraft gegen Null abfällt. Der benötigte Zeitversatz ergibt sich aus der Anwendung. Mehrere Minuten muss die Klebekraft für ein Manipulieren des Filters zur Verfügung stehen, danach soll sie gegen Null abfallen. Zumindest zum Endzeitpunkt der Bebrütung sollte der Fixierrand des Deckels wieder vom Filter lösbar sein. Der Endzeitpunkt der Bebrütung liegt im Fall der klassischen Mikrobiologie vor, wenn mit dem Auge sichtbare Kolonien nach ca. einem Tag auftreten. Die Bebrütungsdauer ist in der Regel in Normen festgelegt und kann bis zu mehreren Tagen andauern. Im Fall von Schnelltests, die Keime und Mikrokolonien schon vorher nachweisen können, sollte die Klebekraft schon spätestens nach ca. 1h soweit abgefallen sein, dass sich der Filter zerstörungsfrei vom Fixierrand des Deckels lösen lässt.

Gemäß einer bevorzugten Ausführungsform der Erfindung wird dieses zeitversetzte Klebe-Löse-Verhalten durch eine Zwischenschicht einer wasserlöslichen Verbindung unter der Kleberschicht verwirklicht. Diese lösbare Schicht wird zwischen Fixierrandoberfläche und Kleberschicht dergestalt aufgebracht, dass die gesamte Klebefläche sicher von der lösbaren Schicht unterlegt ist und auch noch Kontaktflächen für das Eindringen des zum Lösen benötigten Wassers vorhanden sind. Eine derartige Funktion wird z.B. von Gelatine erfüllt. Das für das Lösen benötigte Wasser wird nach dem Auflegen auf die Agarfläche aus dem Agar diffundieren. Alle verwendeten Chemikalien der löslichen Schicht, wie auch des Klebers dürfen das beabsichtigte Wachstum der Mikroorganismen nicht negativ beeinflussen.

Die wasserlösliche Zwischenschicht kann auch am Deckelrand und die Haftschicht am Filterrand angeordnet sein.

Gemäß einer weiteren bevorzugten Ausführungsform der Erfindung wird der Fixierrand von einer freien Stirnfläche einer ringförmigen Innenwandung gebildet, die an der dem Unterteil zugewandten Deckelinnenfläche angeordnet ist und in das Unterteil hineinragt. Die ringförmige Innenwandung stellt zum einen den notwendigen Abstand zwischen Filter und Deckel sicher und zum anderen ist ihr Durchmesser in der Dimension des Filterdurchmessers so gestaltet, dass der Filter nur an seinem außen liegenden Rand mit der Haftschicht der Klebehaftung in Berührung kommt, wobei der Rand außerhalb der benutzten Filterfläche angeordnet ist.

Gemäß einer weiteren bevorzugten Ausführungsform ist die ringförmige Innenwandung so ausgeführt dass die Fixierfläche nicht planar sondern zu zwei vorbestimmten Seiten hin konkav gewölbt ist, so dass in der Anwendung der Abnahme des Filters von der Filterunterstützung eine dieser 2 Seiten zuerst gelöst wird und damit das nach der Vakuumfiltration verbleibende Restvakuum noch gebrochen wird und der Filter damit leichter abnehmbar ist. Auch bei dem Auflegen des Filters auf die Agaroberfläche ergibt sich damit ein positiver Effekt, da damit der Einschluss von Luftblasen weiter vermindert wird.

Das Unterteil der Nährmitteleinheit ist schalenförmig ausgebildet und weist einen Nährboden auf, auf dessen dem Deckel zugewandter Oberseite der am Deckel haftende Filter ablegbar ist. Nach Aufsetzen des Deckels auf das Unterteil liegen der Filter und die Oberseite des Nährbodens eben und abstandsfrei aneinander. Das Unterteil kann beispielsweise eine Petrischale sein, die mit Nährmedium gefüllt ist. Als Nährmedien kommen Agar basierte Medien sowie auch Kartonscheiben gefüllt mit wässrigen Nährmedien in Betracht.

Gemäß einer weiteren bevorzugten Ausführungsform der Erfindung ist die Oberseite des Nährbodens konvex gewölbt.

Dadurch, dass der Nährboden bzw. der Agar konvex gewölbt, d.h. in der Mitte höher ist, hat der Filter bzw. die Membran beim Aufsetzen zuerst mittig Kontakt, sodass beim weiteren Ablegen die Luft nach außen entweichen kann und sich keine Luftblasen bilden. Dies ist wünschenswert, da Luftblasen zwischen Filter und Nährboden sonst eingeschlossen werden könnten, was zu einer Wachstumshemmung führen könnte.

Nach Aufsetzen des Deckels auf das Unterteil liegen der Filter und die Oberseite des Nährbodens eben und abstandsfrei aneinander.

Nach einer weiteren bevorzugten Ausführungsform der Erfindung ist konzentrisch zur Außenwandung des Unterteils ein den Nährboden seitlich in einem unteren Bereich abstützender Stützring angeordnet, dessen Innendurchmesser etwa dem Außendurchmesser des Filters entspricht.

Durch den Stützring wird weniger Agar eingesetzt, da nur die Filterfläche mit Agar unterlegt ist. Der Verstärkungsring hat weiter den Vorteil, dass die Oberfläche des Nährbodens bzw. des Agarbodens sich leichter wölben lässt. Diese Ausführungsform stellt auch sicher, dass durch den vermiedenen Kontakt des Klebers zum Agar, die Klebstoffeigenschaften durch den Agar verändert werden können.

Gemäß einer weiteren bevorzugten Ausführungsform der Erfindung ist der Stützring so positioniert und ausgebildet, dass er durch Kontakt oder Verschluss gegenüber der Haftschicht des Deckels eine etwaige Veränderung der Klebeeigenschaften während der Lagerung, beispielsweise durch Austrocknung, vermindert.

Nach einer weiteren bevorzugten Ausführungsform der Erfindung ist der Deckelinnendurchmesser der Außenwandung des Deckels so gewählt, dass er größer ist als der Außendurchmesser der Filterunterstützung der Filtrationsvorrichtung. Bevorzugt ist der Deckelinnendurchmesser der Außenwandung des Deckels nur wenig größer als der Außendurchmesser der Filterunterstützung. Somit wird der Deckel beim Aufsetzen auf den Filter durch die Filterunterstützung geführt, bevor der Filter bzw. der Deckel mit der Klebehaftung in Kontakt kommt. Die richtige Positionierung des Filters im Deckel wird dadurch sichergestellt.

In einer weiteren bevorzugten Ausführungsform weist die ringförmige Innenwandung des Deckels einen Innenradius auf, der größer oder gleich dem Dichtungsinnenrand des Filtrationstrichters ist. Somit wird während der Positionierung sichergestellt, dass keine Filtrationsfläche überdeckt und damit potenziell filtrierte Keime am Wachstum gehindert werden. Da handelsübliche Filter (vorgegeben durch internationale Normhinweise und -vorschläge) einen Gesamtdurchmesser von 47 mm haben, werden die handelsüblichen Filtrationstrichter in ähnlichen Durchmessern angeboten, womit diese erfindungsgemäße Ausführungsform zum großen Teil auch für alle gängigen Filtrationstrichter angeboten werden kann.

Gemäß einer weiteren bevorzugten Ausführungsform der Erfindung ist die Klebehaftung zwischen Fixierrand und Filter stellenweise unterbrochen. In einer bevorzugten Ausführungsform weist der Fixierrand bzw. die ringförmige Innenwandung mindestens eine Aussparung auf, durch die ein Luftaustausch stattfinden kann. Es ist aber auch möglich, beispielsweise durch zwei Aussparungen die ringförmige Innenwandung in mindestens zwei Innenwandungssegmente mit Stirnflächen in Form von Kreisbogensegmenten variabler Länge zu unterteilen. Diese kreisbogensegmentförmigen Stirnflächen, die in dieser Ausführungsform den Fixierrand bilden, sind über die Haftschicht der Klebehaftung auf dem außen liegenden Rand des Filters fixierbar, wobei der Rand außerhalb der benutzten Filterfläche angeordnet ist. Die Klebehaftung kann auf den kreisbogensegmentförmigen Stirnflächen oder am korrespondierenden Rand des Filters angeordnet sein.

In einer weiteren bevorzugten Ausführungsform sind die mindestens zwei Aussparungen so breit gestaltet, dass der Fixierrand bzw. die ringförmige Innenwandung auf mindestens zwei Fixierstifte reduziert ist, die den Fixierrand bilden und deren von der Deckelinnenfläche abgewandte, freie Enden über die Haftschicht der Klebehaftung auf dem außen liegenden Rand des Filters fixierbar sind, wobei der Rand außerhalb der benutzten Filterfläche angeordnet ist. In einer besonders bevorzugten Ausführungsform sind nur die freien Enden der Fixierstifte mit der Haftschicht beaufschlagt. Durch diese beiden bevorzugten Ausführungsformen ist nicht nur eine Einsparung an dem die ringförmige Innenwandung bildenden Material realisierbar, sondern es ist auch ein Luftaustausch gewährleistet.

Es ist aber auch möglich, den Deckel zur Gewährleistung eines Luftaustausches mit einer wiederverschließbaren Begasungsöffnung zur Begasung der Filteroberfläche auszubilden, um für aerobe Keime einen ausreichenden Luftaustausch und damit die Sauerstoffversorgung während der Inkubation zu gewährleisten.

Vorzugsweise sind die Filter als Membranfilter ausgebildet.

Die weitere Aufgabe bezüglich des Verfahrens wird in Verbindung mit dem Oberbegriff des Anspruches 23 dadurch gelöst, dass der Deckel auf den auf der Filterunterstützung liegenden Filter so aufgesetzt wird, dass ein in dem Deckel angeordneter Fixierrand mit einem Rand des Filters über eine Klebehaftung verbunden wird, und dass der Deckel mit dem anhaftenden Filter von der Filterunterstützung abgehoben und auf das schalenförmige Unterteil der Nährmedieneinheit so aufgesetzt wird, dass die dem Deckel abgewandte Unterseite des Filters auf der dem Deckel zugewandten Oberseite des Nährbodens aufliegt.

Dadurch, dass der Filter mit dem Deckel verhaftet ist und der Deckel mit dem Filter von der Filterunterstützung abgehoben und auf die Oberseite des im Nährmedienbehälter angeordneten Nährbodens aufgesetzt wird, werden zusätzliche Schritte oder Vorrichtungen vermieden und der Filter kann ohne ein zusätzliches Werkzeug in das schalenförmige Unterteil der Nährmedieneinheit verbracht werden. Es können einfache, herkömmliche Filtrationsvorrichtungen verwendet werden. Soweit die Klebehaftung am Deckel angeordnet ist, ist nicht einmal eine Modifikation der Filter notwendig.

Damit ergibt sich ein einfaches und kostengünstiges Manipulieren des Filters mit der aufgebrachten Probe.

Gemäß einer bevorzugten Ausführungsform der Erfindung wird eine an den Fixierrand des Deckels aufgebrachte wasserlösliche Zwischenschicht aufgelöst und der Deckel kann nach einer Inkubation wieder von dem Filter gelöst werden.

Weitere Merkmale der Erfindung ergeben sich aus der nachfolgenden ausführlichen Beschreibung und den beigefügten Zeichnungen, in denen bevorzugte Ausführungsformen der Erfindung beispielhaft veranschaulicht sind.

### Kurzbeschreibungen der Zeichnungen

In den Zeichnungen zeigen:
- Figur 1:: eine Seitenansicht einer Filtrationsvorrichtung im Schnitt,
- Figur 2:: eine Seitenansicht des Vorrichtungsunterteiles von Figur 1 mit Filter und aufgesetztem Deckel einer Nährmedieneinheit im Schnitt,
- Figur 3:: eine Seitenansicht im Schnitt eines Unterteils einer Nährmedieneinheit mit aufgesetztem Deckel und eingebrachtem Filter,
- Figur 4:: eine Seitenansicht eines Filters mit einer Haftschicht im Schnitt und vergrößerter Darstellung,
- Figur 5:: eine Seitenansicht des Deckels von Figur 3 mit anhaftendem Filter im Schnitt und vergrößerter Darstellung,
- Figur 6:: eine Seitenansicht eines Unterteils einer Nährmedieneinheit mit konvex gewölbtem Nährboden und Stützring im Schnitt und vergrößerter Darstellung,
- Figur 7:: eine Untersicht auf den Deckel von Figur 5 mit einer Haftschicht in vergrößerter Darstellung,

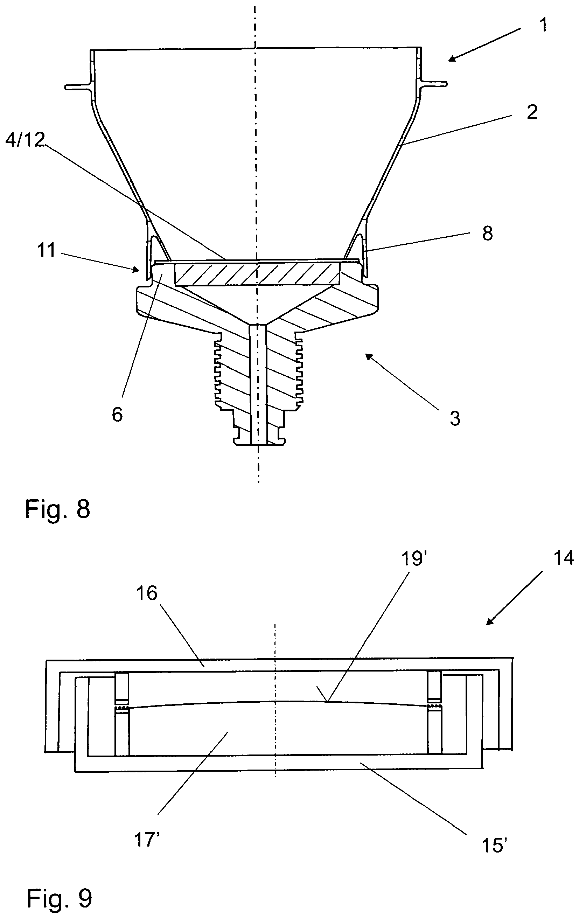

- Figur 8:: eine Seitenansicht einer weiteren Filtrationsvorrichtung im Schnitt,
- Figur 9:: eine Seitenansicht einer Nährmedieneinheit ohne Filter im Schnitt,
- Figur 10:: eine Untersicht auf einen Deckel entsprechend Figur 5 in vergrößerter Darstellung mit Fixierstiften,
- Figur 11:: eine Untersicht auf einen Deckel entsprechend Figur 5 in vergrößerter Darstellung mit Innenwandungssegmenten und
- Figur 12:: eine Seitenansicht eines Deckels mit wasserlöslicher Zwischenschicht, Haftschicht und anhaftendem Filter im Schnitt und Ausriss in vergrößerter Darstellung.

### Beschreibung der Ausführungsbeispiele

Eine Filtrationsvorrichtung 1 besteht im Wesentlichen aus einem Aufsatz 2, einem Vorrichtungsunterteil 3 und einem Filter 4.

Das Vorrichtungsunterteil 3 weist eine Filterunterstützung 5 auf, auf der der Filter 4 abgelegt ist. Das Vorrichtungsunterteil 3 weist weiterhin einen Absatz 6 zur Aufnahme des Aufsatzes 2 auf.

Der Aufsatz 2 ist als ein Trichter 7 ausgebildet, der an seinem in vertikaler Richtung unterem Ende einen zylindrischen Ansatz 8 aufweist, der mit seiner inneren Mantelfläche 9 über den Absatz 6 des Unterteils 3 greift. Im Innenbereich des zylindrischen Ansatzes 8 weist der Trichter 7 eine Auslassöffnung 10 auf, die als Anschlag gegenüber dem Vorrichtungsunterteil 3 bzw. gegenüber dem auf dem Unterteil 3 angeordneten Filter 4 dient, auf. Der Aufsatz 2 bzw. der Trichter 7 kann gegenüber dem Unterteil 3 über eine Rastung 11 verrastet werden.

Der Filter 4 besteht aus einer Membran 12 mit einem Filterrand 13, der außerhalb der Auslassöffnung 10 angeordnet ist.

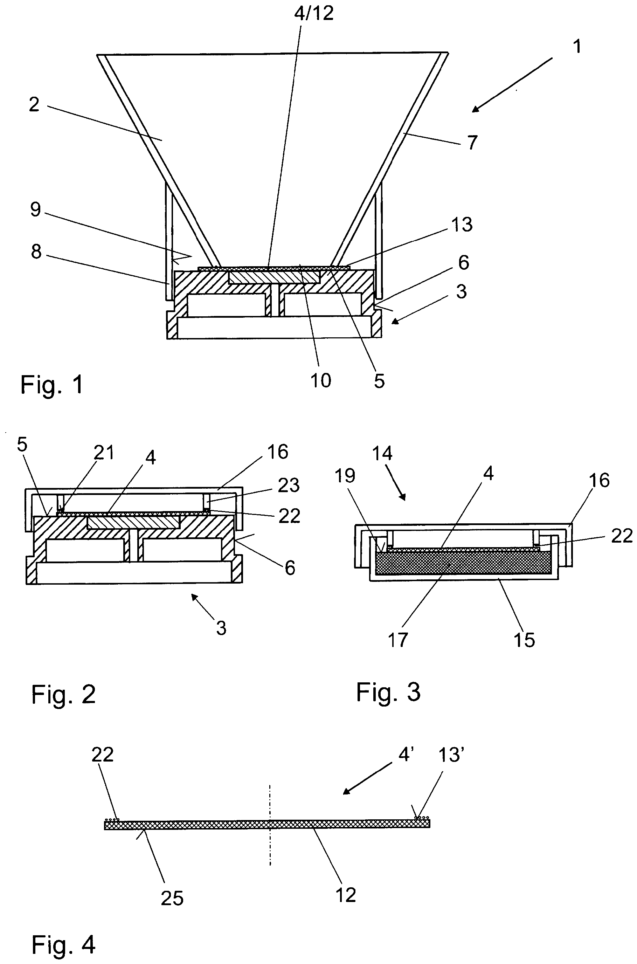

Nach der Ausführungsform von Figur 4 ist der Filterrand 13 des Filters 4' mit einer Klebehaftung 22 versehen.

Nach dem Filtrieren der Probe wird der Filter 4, 4' in eine Nährmedieneinheit 14 eingebracht. Die Nährmedieneinheit 14 besteht aus einem schalenförmigen Unterteil 15, das als eine Petrischale oder eine Agarschale ausgebildet sein kann, und aus einem Deckel 16. In dem Unterteil 15 ist ein Nährboden 17, beispielsweise aus Agar, angeordnet.

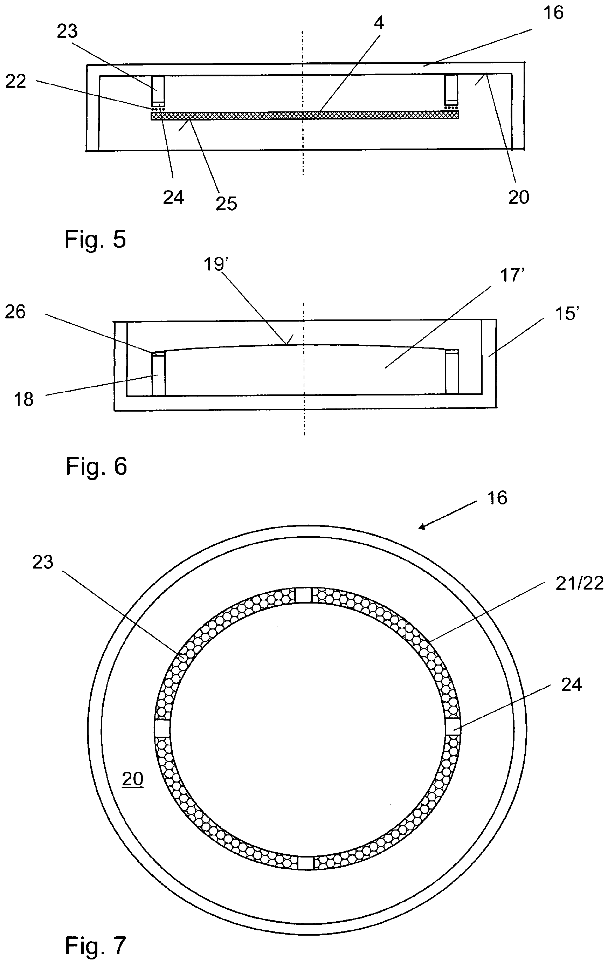

Entsprechend dem Ausführungsbeispiel von Figur 6 weist das Unterteil 15' einen Stützring 18 auf, der den Nährboden 17' seitlich abstützt. Im unbelasteten Zustand ist die Oberseite 19, 19' des Nährbodens 17, 17', die in vertikaler Richtung oben liegt, konvex gewölbt, sodass der Nährboden 17, 17' zur Mitte hin etwas dicker ist.

Der Deckel 16 ist auf das Unterteil 15, 15' der Nährmedieneinheit 14 aufsetzbar. An seiner dem Unterteil 15, 15' zugewandten Deckelinnenfläche 20 weist der Deckel 16 einen in das Unterteil 15, 15' hineinragenden Fixierrand 21 auf, der zur Entnahme des Filters 4, 4' aus der Filtrationsvorrichtung 1 mit dem Filterrand 13 des Filters 4, 4' über die Klebehaftung 22 mit dem Filter 4, 4' verbindbar ist. Der Fixierrand 21 wird von einer freien Stirnfläche einer an der Deckelinnenfläche 20 angeordneten ringförmigen Innenwandung 23 gebildet.

Entsprechend Figur 7 ist die Klebehaftung 22 als eine Haftschicht 27 aus einem geeigneten Kleber am Fixierrand 21 des Deckels 16 angeordnet. Es ist aber auch möglich, die Haftschicht gemäß Figur 4 am Filterrand 13' anzuordnen.

Die Klebehaftung 22 zwischen Fixierrand 21 und Filter 4 ist stellenweise zur Gewährleistung eines Luftaustausches unterbrochen. Hierzu weist die Innenwandung 23 des Deckels 16 an ihrer Stirnfläche bzw. am Fixierrand 21 Aussparungen 24 auf. Auch ist es möglich, am Stützring 18 Aussparungen 26 anzuordnen. Die Haftschicht der Klebehaftung 22 ist aus einem Kleber PSA-Dispersionsklebstoff oder aus Acrylat-Copolymer-Mikrokugeln ausgebildet.

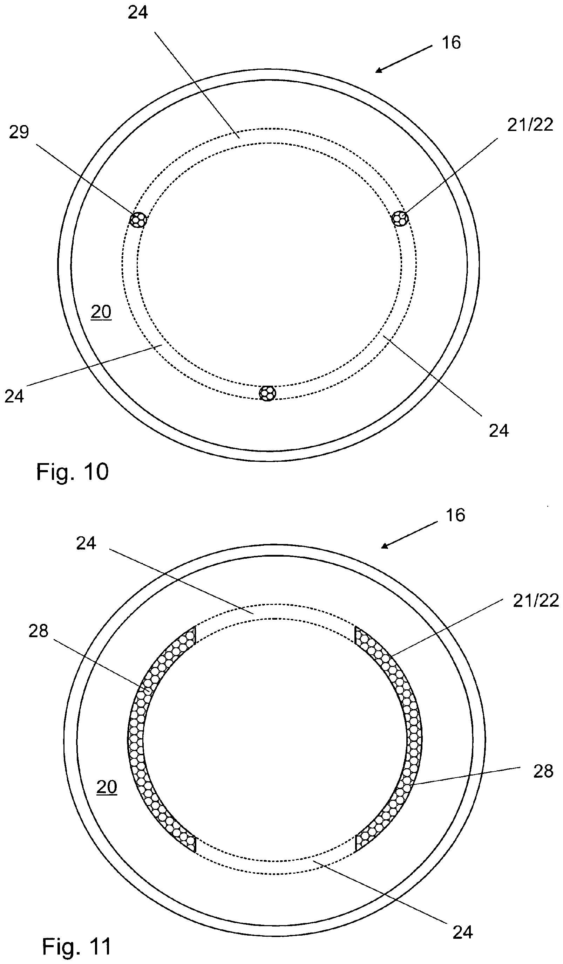

In einer bevorzugten Ausführungsform gemäß Figur 10 sind die Aussparungen 24 so breit gestaltet, dass die ringförmige Innenwandung 23 bzw. der Fixierrand 21 auf mindestens zwei Fixierstifte 29 reduziert ist, deren von der Deckelinnenfläche 20 abgewandte, freie Enden über die Haftschicht der Klebehaftung 22 auf dem außen liegenden Rand 13 des Filters fixierbar sind, wobei der Rand 13 außerhalb der benutzten Filterfläche angeordnet ist. Die freien Enden sind hierbei mit der Haftschicht bzw. Klebehaftung 22 beaufschlagt. Bei einer nicht dargestellten alternativen Ausführungsform weisen die Fixierstifte 29 an ihren freien Enden keine Haftschicht auf. Die Haftschicht ist hierbei am Filterrand 13 angeordnet. Aus Stabilitätsgründen für die Haftung zwischen Filter 4 und Deckel 16 sind mindestens zwei Fixierstifte 29 vorgesehen. Figur 10 zeigt drei Fixierstifte 29, wobei diese bevorzugt äquidistant auf einem gedachten Kreisumfang angeordnet sind.

Das Ausführungsbeispiel von Figur 11 zeigt eine alternative Ausführungsform eines Deckels 16, dessen Fixierrand 21 von zwei Innenwandungssegmenten 28 mit dazwischen liegenden Aussparungen 24 gebildet wird.

Zum Einbringen des als Membranfilter ausgebildeten Filters 4 in das Unterteil 15, 15' der Nährmedieneinheit 14 wird nach dem Filtrieren der Aufsatz 2 von der Filterunterstützung 5 des Vorrichtungsunterteils 3 abgehoben. Anschließend wird der Deckel 16 von dem Unterteil 15, 15' der Nährmedieneinheit 14 abgenommen und auf den auf der Filterunterstützung 5 liegenden Filter 4 so aufgesetzt, dass der in dem Deckel 16 angeordnete Fixierrand 21 mit dem Filterrand 13 des Filters 4 über die Klebehaftung 22 verbunden wird und der Filter 4 an dem Deckel 16 anhaftet. Der Deckel 16 wird sodann mit dem Filter 4 von der Filterunterstützung 5 abgehoben und auf das Unterteil 15, 15' der Nährmedieneinheit 14 aufgesetzt. Dabei wird der Deckel 16 mit der Unterseite 25 des Filters 4 auf die Oberseite 19, 19' des Nährbodens 17, 17' aufgelegt.

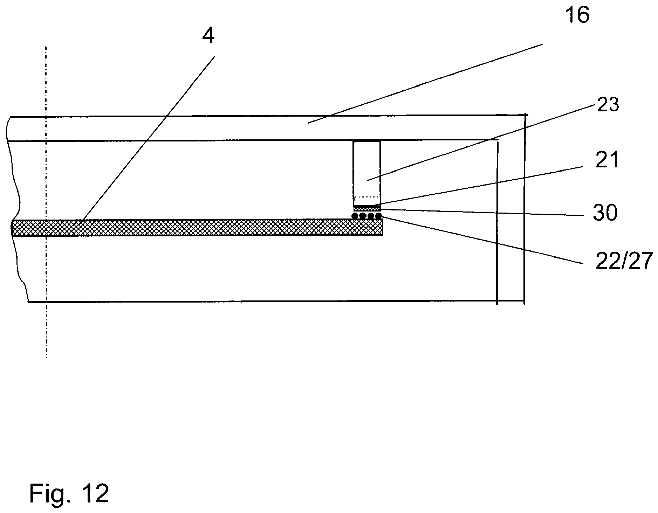

Entsprechend dem Ausführungsbeispiel von Figur 12 ist zwischen der Haftschicht 27 der Klebehaftung 22 und dem Fixierrand 21 des Deckels 16 eine wasserlösliche Zwischenschicht 30 aus Gelatine aufgebracht.

## Patentansprüche

1. Nährmedieneinheit (14) zur Aufnahme eines Filters (4, 4') einer Filtrationsvorrichtung (1) mit einem mit Nährmedium gefüllten Unterteil (15, 15') und einem Deckel (16),
**dadurch gekennzeichnet,**
**dass** der Deckel (16) einen in das Unterteil (15, 15') hineinragenden Fixierrand (21) aufweist, der zur Entnahme des Filters (4) aus der Filtrationsvorrichtung (1) mit einem Rand (13, 13') des Filters (4, 4') über eine Klebehaftung (22) mit dem Filter (4, 4') verbindbar ist.

2. Nährmedieneinheit nach Anspruch 1,
**dadurch gekennzeichnet,**
**dass** die Klebehaftung (22) temporär oder reversibel ausgebildet ist.

3. Nährmedieneinheit nach Anspruch 1 oder 2,
**dadurch gekennzeichnet,**
**dass** der Fixierrand (21) des Deckels (16) eine Haftschicht (27) aus einem geeigneten Kleber aufweist.

4. Nährmedieneinheit nach Anspruch 2 oder 3,
**dadurch gekennzeichnet,**
**dass** der Kleber über eine wasserlösliche Zwischenschicht (30) an den Fixierrand des Deckels aufgebracht ist.

5. Nährmedieneinheit nach Anspruch 1,
**dadurch gekennzeichnet,**
**dass** der Filterrand (13) des Filters (4') eine Haftschicht (27) aus einem geeigneten Kleber aufweist.

6. Nährmedieneinheit nach Anspruch 5,
**dadurch gekennzeichnet,**
**dass** der Fixierrand (21) des Deckels (16) eine wasserlösliche Schicht (30) aufweist.

7. Nährmedieneinheit nach einem der Ansprüche 3 bis 6,
**dadurch gekennzeichnet,**
**dass** die Haftschicht (27) aus einem PSA-Dispersionsklebstoff oder aus Acrylat-Copolymer-Mikrokugeln ausgebildet ist.

8. Nährmedieneinheit nach einem der Ansprüche 1 bis 7,
**dadurch gekennzeichnet,**
**dass** der Fixierrand (21) von einer freien Stirnfläche einer an der dem Unterteil (15, 15') zugewandten Deckelinnenfläche (20) angeordneten ringförmigen Innenwandung (23) gebildet wird.

9. Nährmedieneinheit nach Anspruch 8,
**dadurch gekennzeichnet,**
**dass** die Fixierfläche des Fixierrandes (21) zu zwei vorbestimmten Seiten hin konkav gewölbt ist.

10. Nährmedieneinheit nach einem der Ansprüche 1 bis 9,
**dadurch gekennzeichnet,**
**dass** das Unterteil (15, 15') schalenförmig ausgebildet ist und einen Nährboden (17, 17') aufweist, auf dessen dem Deckel (16) zugewandter Oberseite (19, 19') der am Deckel (16) haftende Filter (4, 4') ablegbar ist.

11. Nährmedieneinheit nach Anspruch 10,
**dadurch gekennzeichnet,**
**dass** die Oberseite (19, 19') des Nährbodens (17, 17') konvex gewölbt ist.

12. Nährmedieneinheit nach einem der Ansprüche 8 bis 9,
**dadurch gekennzeichnet,**
**dass** nach dem Aufsetzen des Deckels (16) auf das Unterteil (15, 15') der Filter (4) und die Oberseite (19, 19') des Nährbodens (17, 17') eben und abstandsfrei aneinanderliegen.

13. Nährmedieneinheit nach einem der Ansprüche 10 bis 12,
**dadurch gekennzeichnet,**
**dass** im Unterteil (15') konzentrisch zur Außenwandung ein den Nährboden (17') seitlich in einem unteren Bereich abstützender Stützring (18) angeordnet ist, dessen Innendurchmesser etwa dem Außendurchmesser des Membranfilters (4, 4') entspricht.

14. Nährmedieneinheit nach Anspruch 13,
**dadurch gekennzeichnet,**
**dass** der Stützring (18) so positioniert und ausgebildet ist, dass er durch Kontakt oder Verschluss gegenüber der Haftschicht (27) des Deckels (16) eine etwaige Veränderung der Klebeeigenschaften während der Lagerung vermindert.

15. Nährmedieneinheit nach einem der Ansprüche 1 bis 14,
**dadurch gekennzeichnet,**
**dass** der Deckelinnendurchmesser der Außenwandung des Deckels (16) so gewählt ist, dass er größer ist, als der Außendurchmesser einer Filterunterstützung der Filtrationsvorrichtung (1).

16. Nährmedieneinheit nach Anspruch 15,
**dadurch gekennzeichnet,**
**dass** der Deckelinnendurchmesser der Außenwandung des Deckels (16) nur wenig größer ist, als der Außendurchmesser der Filterunterstützung.

17. Nährmedieneinheit nach einem der Ansprüche 1 bis 16,
**dadurch gekennzeichnet,**
**dass** die Klebehaftung (22) zwischen Fixierrand (21) und Filter (4) stellenweise zur Gewährleistung eines Luftaustausches unterbrochen ist.

18. Nährmedieneinheit nach einem der Ansprüche 1 bis 17,
**dadurch gekennzeichnet,**
**dass** der Fixierrand (21) des Deckels (16) durch mindestens eine Aussparung zur Gewährleistung eines Luftaustausches unterbrochen ist.

19. Nährmedieneinheit nach einem der Ansprüche 1 bis 18,
**dadurch gekennzeichnet,**
**dass** der Fixierrand (21) des Deckels (16) auf mindestens zwei Fixierstifte (29) reduziert ist, über welche der Filter (4) mit dem Deckel (16) verbindbar ist.

20. Nährmedieneinheit nach einem der Ansprüche 1 bis 16,
**dadurch gekennzeichnet,**
**dass** der Deckel (16) zur Gewährleistung eines Luftaustausches eine wiederverschließbare Begasungsöffnung aufweist.

21. Nährmedieneinheit nach einem der Ansprüche 1 bis 20,
**dadurch gekennzeichnet,**
**dass** der Nährboden (17, 17') aus Agar ausgebildet ist.

22. Nährmedieneinheit nach einem der Ansprüche 1 bis 21,
**dadurch gekennzeichnet,**
**dass** der Filter (4) als ein Membranfilter ausgebildet ist.

23. Verfahren zur mikrobiologischen Untersuchung flüssiger Proben nach vorangegangener Filtration, bei dem ein Filter (4, 4') von einer Filterunterstützung (5) abgehoben und auf einer Oberfläche (19, 19') eines in einem Unterteil (15, 15') einer Nährmedieneinheit (14) angeordneten Nährbodens (17) abgelegt und das Unterteil (15, 15') von einem Deckel (16) abgedeckt wird,
**dadurch gekennzeichnet,**
**dass** der Deckel (16) auf den auf der Filterunterstützung (5) liegenden Filter (4, 4') so aufgesetzt wird, dass ein in dem Deckel (16) angeordneter Fixierrand (21) mit einem Rand (13) des Filters (4, 4') über eine Klebehaftung (22) verbunden wird, und
**dass** der Deckel (16) mit dem anhaftenden Filter (4, 4') von der Filterunterstützung (5) abgehoben und auf das schalenförmige Unterteil (15, 15') der Nährmedieneinheit (14) so aufgesetzt wird, dass die dem Deckel (16) abgewandte Unterseite (25) des Filters (4, 4') auf der dem Deckel (16) zugewandten Oberseite (19, 19') des Nährbodens (17, 17') aufliegt.

24. Verfahren nach Anspruch 23,
**dadurch gekennzeichnet,**
**dass** eine an den Fixierrand des Deckels aufgebrachte wasserlösliche Zwischenschicht (30) aufgelöst und der Deckel nach einer Inkubation wieder von dem Filter gelöst wird.

25. Verfahren nach Anspruch 23 oder 24,
**dadurch gekennzeichnet,**
**dass** durch mindestens eine Aussparung (24) des Fixierrandes (21) ein Luftaustausch erfolgt.

## Claims

1. Nutrient medium unit (14) for receiving a filter (4, 4') of a filtration device (1) with a lower part (15, 15'), which is filled with a nutrient medium, and a cover (16), **characterised in that** the cover (16) has a fixing edge (21) which projects into the lower part (15, 15') and which for removal of the filter (4) from the filtration device (1) is connectible with an edge (13, 13') of the filter (4, 4') by way of an adhesive bond (22) with the filter (4, 4').

2. Nutrient medium unit according to claim 1, **characterised in that** the adhesive bond (22) is formed to be temporary or reversible.

3. Nutrient medium unit according to claim 1 or 2, **characterised in that** the fixing edge (21) of the cover (16) has an adhesion layer (27) of a suitable adhesive.

4. Nutrient medium unit according to claim 2 or 3, **characterised in that** the adhesive is applied to the fixing edge of the cover by way of a water-soluble intermediate layer (30).

5. Nutrient medium unit according to claim 1, **characterised in that** the filter edge (13) of the filter (4') has an adhesive layer (27) of a suitable adhesive.

6. Nutrient medium unit according to claim 5, **characterised in that** the fixing edge (21) of the cover (16) has a water-soluble layer (30).

7. Nutrient medium unit according to any one of claims 3 to 6, **characterised in that** the adhesive layer (27) is formed from a PSA dispersion adhesive or from acrylate copolymer microspheres.

8. Nutrient medium unit according to any one of claims 1 to 7, **characterised in that** the fixing edge (21) is formed by a free end surface of an annular inner wall (23) arranged at the cover inner surface (20) facing the lower part (15, 15').

9. Nutrient medium unit according to claim 8, **characterised in that** the fixing surface of the fixing edge (21) is concavely curved towards two predetermined sides.

10. Nutrient medium unit according to any one of claims 1 to 9, **characterised in that** the lower part (15, 15') is of basin-shaped construction and has a nutrient base (17, 17') on the upper side (19, 19'), which faces the cover (16), of which the filter (4, 4') adhering to the cover (16) can be placed.

11. Nutrient medium unit according to claim 10, **characterised in that** the upper side (19, 19') of the nutrient base (17, 17') is convexly curved.

12. Nutrient medium unit according to one of claims 8 and 9, **characterised in that** after placing the cover (16) on the lower part (15, 15') the filter (4) and the upper side (19, 19') of the nutrient base (17, 17') rest one against the other flatly and without spacing.

13. Nutrient medium unit according to any one of claims 10 to 12, **characterised in that** a support ring (18), which supports the nutrient base (17') laterally in a lower region and the inner diameter of which approximately corresponds with the outer diameter of the membrane filter (4, 4'), is arranged in the lower part (15') concentrically with the outer wall.

14. Nutrient medium unit according to claim 13, **characterised in that** the support ring (18) is so positioned and constructed that through contact or sealing relative to the adhesive layer (27) of the cover (16) it reduces possible change in the adhesion characteristics during mounting.

15. Nutrient medium unit according to any one of claims 1 to 14, **characterised in that** the cover inner diameter of the outer wall of the cover (16) is selected so that it is larger than the outer diameter of a filter support of the filtration device (1).

16. Nutrient medium unit according to claim 15, **characterised in that** the cover inner diameter of the outer wall of the cover (16) is only slightly larger than the outer diameter of the filter support.

17. Nutrient medium unit according to any one of claims 1 to 16, **characterised in that** the adhesive bond (22) between fixing edge (21) and filter (4) is locally interrupted to ensure air exchange.

18. Nutrient medium unit according to any one of claims 1 to 17, **characterised in that** the fixing edge (21) of the cover (16) is interrupted by at least one cut-out to ensure air exchange.

19. Nutrient medium unit according to any one of claims 1 to 18, **characterised in that** the fixing edge (21) of the cover (16) is reduced to at least two fixing pins (29) by way of which the filter (4) is connectible with the cover (16).

20. Nutrient medium unit according to any one of claims 1 to 16, **characterised in that** the cover (16) has a reclosable gassing opening to ensure air exchange.

21. Nutrient medium unit according to any one of claims 1 to 20, **characterised in that** the nutrient base (17, 17') is formed from agar.

22. Nutrient medium unit according to any one of claims 1 to 21, **characterised in that** the filter (4) is constructed as a membrane filter.

23. Method for microbiological investigation of liquid samples after preceding filtration, in which a filter (4, 4') is lifted off a filter support (5) and deposited on a surface (19, 19') of a nutrient base (17) arranged in a lower part (15, 15') of a nutrient medium unit (14) and the lower part (15, 15') is covered by a cover (16), **characterised in that** the cover (16) is so placed on the filter (4, 4') lying on the filter support (5) that a fixing edge (21) arranged in the cover (16) is connected with an edge (13) of the filter (4, 4') by way of an adhesive bond (22) and
the cover (16) with the adhering filter (4, 4') is lifted off the filter support (5) and so placed on the basin-shaped lower part (15, 15') of the nutrient medium unit (14) that the lower side (25), which is remote from the cover (16), of the filter (4, 4') rests on the upper side (19, 19'), which faces the cover (16), of the nutrient base (17, 17').

24. Method according to claim 23, **characterised in that** a water-soluble intermediate layer (30) applied to the fixing edge of the cover is released and the cover is detached again from the filter after an incubation.

25. Method according to claim 23 or 24, **characterised in that** air exchange takes place through at least one cut-out (24) of the fixing edge (21).

## Revendications

1. Unité de milieu nutritif (14) destinée à loger un filtre (4, 4') d'un dispositif de filtration (1), et comprenant une partie inférieure (15, 15') remplie d'un milieu nutritif et un couvercle (16), **caractérisée en ce**
**que** le couvercle (16) comprend un bord de fixation (21) qui fait saillie à l'intérieur de la partie inférieure (15, 15') et qui, pour prélever le filtre (4) du dispositif de filtration (1), peut être relié au filtre (4, 4') par un bord (13, 13') du filtre (4, 4') au moyen d'une liaison adhésive (22).

2. Unité de milieu nutritif selon la revendication 1,
**caractérisée en ce que**
la liaison adhésive (22) est temporaire ou réversible.

3. Unité de milieu nutritif selon la revendication 1 ou 2,
**caractérisée en ce que**
le bord de fixation (21) du couvercle (16) comprend une couche adhésive (27) faite d'un adhésif adapté.

4. Unité de milieu nutritif selon la revendication 2 ou 3,
**caractérisée en ce que**
l'adhésif est appliqué sur le bord de fixation du couvercle au moyen d'une couche intermédiaire (30) soluble dans l'eau.

5. Unité de milieu nutritif selon la revendication 1,
**caractérisée en ce que**
le bord de filtre (13) du filtre (4') comprend une couche d'adhésif (27) faite d'un adhésif adapté.

6. Unité de milieu nutritif selon la revendication 5,
**caractérisée en ce que**
le bord de fixation (21) du couvercle (16) comprend une couche (30) soluble dans l'eau.

7. Unité de milieu nutritif selon l'une quelconque des revendications 3 à 6,
**caractérisée en ce que**
la couche adhésive (27) est réalisée à partir d'un adhésif en dispersion sensible à la pression ou de microbilles de copolymère d'acrylate.

8. Unité de milieu nutritif selon l'une quelconque des revendications 1 à 7,
**caractérisée en ce que**
le bord de fixation (21) est formé par une surface frontale libre d'une paroi intérieure (23) annulaire agencée sur la surface intérieure de couvercle (20) tournée vers la partie inférieure (15, 15').

9. Unité de milieu nutritif selon la revendication 8,
**caractérisée en ce que**
la surface de fixation du bord de fixation (21) est courbée de manière concave en direction de deux faces prédéfinies.

10. Unité de milieu nutritif selon l'une quelconque des revendications 1 à 9,
**caractérisée en ce que**
la partie inférieure (15, 15') est en forme de coque et comprend un fond nutritif (17, 17') sur la face supérieure (19, 19') duquel peut être déposé le filtre (4, 4') adhérant au couvercle (16), ladite face étant tournée vers le couvercle (16).

11. Unité de milieu nutritif selon la revendication 10,
**caractérisée en ce que**
la face supérieure (19, 19') du fond nutritif (17, 17') est courbée de manière convexe.

12. Unité de milieu nutritif selon l'une quelconque des revendications 8 à 9,
**caractérisée en ce que**
une fois le couvercle (16) posé sur la partie inférieure (15, 15'), le filtre (4) et la face supérieure (19, 19') du fond nutritif (17, 17') sont juxtaposés de façon plane sans présenter d'écart entre eux.

13. Unité de milieu nutritif selon l'une quelconque des revendications 10 à 12,
**caractérisée en ce que**
une bague d'appui (18), qui soutient le fond nutritif (17') latéralement dans une zone inférieure et dont le diamètre intérieur correspond sensiblement au diamètre extérieur du filtre à membrane (4, 4'), est agencée dans la partie inférieure (15') de manière concentrique par rapport à la paroi extérieure.

14. Unité de milieu nutritif selon la revendication 13,
**caractérisée en ce que**
la bague d'appui (18) est positionnée et conçue de sorte qu'elle diminue une modification éventuelle des propriétés adhésives pendant le stockage, par contact ou obturation vis-à-vis de la couche adhésive (27) du couvercle (16).

15. Unité de milieu nutritif selon l'une quelconque des revendications 1 à 14,
**caractérisée en ce que**
le diamètre intérieur de couvercle de la paroi extérieure du couvercle (16) est sélectionné de manière à être supérieur au diamètre extérieur d'un support de filtre du dispositif de filtration (1).

16. Unité de milieu nutritif selon la revendication 15,
**caractérisée en ce que**
le diamètre intérieur de couvercle de la paroi intérieure du couvercle (16) n'est qu'un peu plus grand que le diamètre extérieur du support de filtre.

17. Unité de milieu nutritif selon l'une quelconque des revendications 1 à 16,
**caractérisée en ce que**
la liaison adhésive (22) entre le bord de fixation (21) et le filtre (4) est interrompue par endroits pour garantir un échange d'air.

18. Unité de milieu nutritif selon l'une quelconque des revendications 1 à 17,
**caractérisée en ce que**
le bord de fixation (21) du couvercle (16) est interrompu par au moins un évidement pour garantir un échange d'air.

19. Unité de milieu nutritif selon l'une quelconque des revendications 1 à 18,
**caractérisée en ce que**
le bord de fixation (21) du couvercle (16) est réduit à au moins deux broches de fixation (29), par l'intermédiaire desquelles le filtre (4) peut être relié au couvercle (16).

20. Unité de milieu nutritif selon l'une quelconque des revendications 1 à 16,
**caractérisée en ce que**
le couvercle (16) comprend une ouverture de dégazage refermable pour garantir un échange d'air.

21. Unité de milieu nutritif selon l'une quelconque des revendications 1 à 20,
**caractérisée en ce que**
le fond nutritif (17, 17') est fait d'agar-agar.

22. Unité de milieu nutritif selon l'une quelconque des revendications 1 à 21,
**caractérisée en ce que**
le filtre (4) est réalisé sous la forme d'un filtre à membrane.

23. Procédé d'analyse microbiologique d'échantillons liquides ayant été précédemment filtrés, selon lequel un filtre (4, 4') est soulevé d'un support de filtre (5) et déposé sur une surface (19, 19') d'un fond nutritif (17) agencé dans une partie inférieure (15, 15') d'une unité de milieu nutritif (14) et la partie inférieure (15, 15') est recouverte par un couvercle (16),
**caractérisé en ce que**
le couvercle (16) est posé sur le filtre (4, 4') situé sur le support de filtre (5), de sorte qu'un bord de fixation (21) agencé dans le couvercle (16) est relié à un bord (13) du filtre (4, 4') au moyen d'une liaison adhésive (22), et
**en ce que** le couvercle (16) auquel adhère le filtre (4, 4') est soulevé du support de filtre (5) et posé sur la partie inférieure (15, 15') en forme de coque de l'unité de milieu nutritif (14), de sorte que la face inférieure (25) du filtre (4, 4') opposée au couvercle (16) repose sur la face supérieure (19, 19') du fond nutritif (17, 17') tournée vers le couvercle (16).

24. Procédé selon la revendication 23,
**caractérisé en ce que**
une couche intermédiaire (30) soluble dans l'eau appliquée sur le bord de fixation du couvercle est dissoute et le couvercle est à nouveau détaché du filtre après une incubation.

25. Procédé selon la revendication 23 ou 24,
**caractérisé en ce que**
un échange d'air se fait à travers au moins un évidement (24) du bord de fixation (21).
